# EUROPEAN PATENT SPECIFICATION

(11) **EP 3 307 718 B1**
(45) Date of publication and mention of the grant of the patent: **30.12.2020**
(21) Application number: 16748346.0
(22) Date of filing: 13.06.2016
(51) Int. Cl.: C07D 249/12

(54) **POLYMORPHS OF A PGES-1 INHIBITING TRIAZOLONE COMPOUND**
POLYMORPHE EINER PGES-1-HEMMENDEN TRIAZOLONVERBINDUNG
POLYMORPHES D'UN COMPOSÉ DE TRIAZOLONE INHIBANT PGES-1

(30) Priority: 12.06.2015 IN 2275MU2015
(43) Date of publication of application: 18.04.2018
(73) Proprietor: Glenmark Pharmaceuticals S.A., 2300 La Chaux-de-Fonds (CH)
(72) Inventor: MUTHUKAMAN, Nagarajan, Erode Tamil Nadu 638151 (IN); GHARAT, Laxmikant Atmaram, Thane (West) Maharashtra 400601 (IN); KADAM, Suresh Mahadev, Thane (West) Maharashtra 400607 (IN); GAVHANE, Sachin, Thane Maharashtra 421204 (IN); KHANDAGALE, Sandeep Bandu, Ahmednagar Maharashtra 413709 (IN); NIRGUDE, Sunil Pandurang, Thane (West) Maharashtra 400607 (IN)
(74) Representative: HGF
(86) International application number: PCT/IB2016/053467
(87) International publication number: WO 2016/199104

(56) References cited:
- WO-A1-2012/087771
- WO-A1-2013/186692
- HILFIKER R (EDITOR) ED - HILFIKER R: "Polymorphism in the Pharmaceutical Industry", 1 January 2006 (2006-01-01), 20060101, PAGE(S) 1 - 19, XP002528052, ISBN: 978-3-527-31146-0 Chapters 1 and 1.4.2
- CAIRA M R: "CRYSTALLINE POLYMORPHISM OF ORGANIC COMPOUNDS", TOPICS IN CURRENT CHEMISTRY, SPRINGER, BERLIN, DE, vol. 198, 1 January 1998 (1998-01-01), pages 163-208, XP001156954, ISSN: 0340-1022, DOI: 10.1007/3-540-69178-2_5
- YU L ED - KWON ICK CHAN ET AL: "AMORPHOUS PHARMACEUTICAL SOLIDS: PREPARATION, CHARACTERIZATION AND STABILIZATION", ADVANCED DRUG DELIVERY REVIEWS, ELSEVIER, AMSTERDAM, NL, vol. 48, no. 1, 16 May 2001 (2001-05-16), pages 27-42, XP009065056, ISSN: 0169-409X, DOI: 10.1016/S0169-409X(01)00098-9

## Description

### TECHNICAL FILED OF THE INVENTION

The present application relates to solid state forms of a triazolone compound which exhibit mPGES-1 enzyme inhibition activity, specifically *N*-(4-chloro-3-(5-oxo-1-(4-(trifluoromethyl)phenyl)-4,5-dihydro-1*H*-1,2,4-triazol-3-yl)benzyl) pivalamide, and process for preparation thereof.

### BACKGROUND OF THE INVENTION

There are many diseases or disorders that are inflammatory in their nature. One of the major problems associated with existing treatments of inflammatory conditions is inadequate efficacy and/or the prevalence of side effects. Inflammatory diseases that affect the population include asthma, inflammatory bowel disease, rheumatoid arthritis, osteoarthritis, rhinitis, conjunctivitis and dermatitis. Inflammation is also a common cause of pain.

The enzyme cyclooxygenase (COX) converts arachidonic acid to an unstable intermediate, prostaglandin H₂ (PGH₂), which is further converted to other prostaglandins, including PGE₂, PGF_{2α}, PGD₂, prostacyclin and thromboxane A₂. These arachidonic acid metabolites are known to have pronounced physiological and pathophysiological activity, including pro-inflammatory effects. The COX enzyme exists in two forms, one that is constitutively expressed in many cells and tissues (COX-1), and another that in mast cells and tissues is induced by pro-inflammatory stimuli, such as cytokines, during an inflammatory response (COX-2).

Among all prostaglandin metabolites, PGE₂ is particularly known to be a strong pro-inflammatory mediator, and is also known to induce fever and pain. Consequently, numerous drugs have been developed with a view to inhibiting the formation of PGE₂, including "NSAIDs" (non-steroidal anti-inflammatory drugs) and "coxibs" (selective COX-2 inhibitors). These drugs act predominantly by inhibition of COX-1 and/or COX-2, thereby reducing the formation of PGE₂. However, the inhibition of COXs has the disadvantage of reducing the formation of all metabolites of PGH₂, thereby decreasing the beneficial properties of some of the metabolites. In view of this, drugs which act by inhibition of COXs are suspected to cause adverse biological effects. For example, the non-selective inhibition of COXs by NSAIDs may give rise to gastrointestinal side-effects and affect platelet and renal function. Even the selective inhibition of COX-2 by coxibs, whilst reducing such gastrointestinal side-effects, is believed to give rise to cardiovascular problems.

A combination of pharmacological, genetic and neutralizing antibody approaches demonstrates the importance of PGE₂ in inflammation. The conversion of PGH₂ to PGE₂ by prostaglandin E synthases (PGES) may, therefore, represent a pivotal step in the propagation of inflammatory stimuli. There are two microsomal prostaglandin E synthases (mPGES-1 and mPGES-2), and one cytosolic prostaglandin E synthase (cPGES). mPGES-1 is an inducible PGES after exposure to pro-inflammatory stimuli. mPGES-1 is induced in the periphery and CNS by inflammation, and represents therefore a target for acute and chronic inflammatory disorders. PGE₂ is a major prostanoid, produced from arachidonic acid liberated by phospholipases (PLAs), which drives the inflammatory processes. Arachidonic acid is transformed by the action of prostaglandin H synthase (PGH synthase, cycloxygenase) into PGH₂ which is a substrate for mPGES-1, the terminal enzyme transforming PGH₂ to the pro-inflammatory PGE₂.

Agents that are capable of inhibiting the action of mPGES-1, and thus reducing the formation of the specific arachidonic acid metabolite PGE₂, are beneficial in the treatment of inflammation. Further, agents that are capable of inhibiting the action of the proteins involved in the synthesis of the leukotrienes are also beneficial in the treatment of asthma and COPD.

Blocking the formation of PGE₂ in animal models of inflammatory pain results in reduced inflammation, pain and fever response (Kojima et. al, The Journal of Immunology 2008, 180, 8361-6; Xu et. al., The Journal of Pharmacology and Experimental Therapeutics 2008, 326, 754-63). In abdominal aortic aneurism, inflammation leads to connective tissue degradation and smooth muscle apoptosis ultimately leading to arotic dilation and rupture. In animals lacking mPGES-1 a slower disease progression and disease severity has been demonstrated (Wang et. al., Circulation, 2008, 117, 1302-1309).

Several lines of evidence indicate that PGE₂ is involved in malignant growth. PGE₂ facilitates tumor progression by stimulation of cellular proliferation and angiogenesis and by modulation of immunosupression. In support of a role for PGE₂ in cancers, genetic deletion of mPGES-1 in mice suppresses intestinal tumourogenesis (Nakanishi et. al., Cancer Research 2008, 68(9), 3251-9). In human beings, mPGES-1 is also upregulated in cancers such as colorectal cancer (Schroder, Journal of Lipid Research 2006, 47, 1071-80).

International Publication Number WO 2013/186692 discloses triazolone compounds of formula (I) (disclosed as formula (III) in the specification of WO 2013/186692) 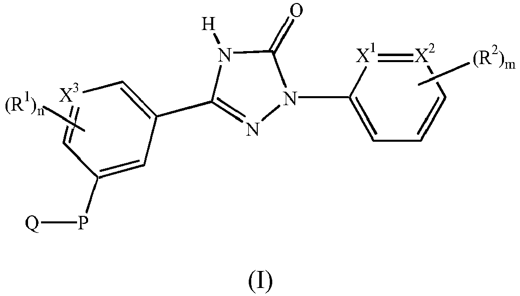 wherein,
X¹, X² and X³ are each independently selected from CH and N;
P is selected from -CH₂NHC(O)- and -CH₂NHS(O)₂-;
Q is selected from C₁₋₈alkyl, haloC₁₋₈alkyl, C₁₋₈alkoxyC₁₋₈alkyl, hydroxyC₁₋₈alkyl, carboxylC₁₋₈alkyl, C₃₋₁₂cycloalkyl, C₆₋₁₄aryl, 3-15 membered heterocyclyl, and 5-14 membered heteroaryl;
each occurrence of R¹ is independently selected from halogen, cyano, C₁₋₈alkyl, C₁₋₈alkoxy, haloC₁₋₈alkyl and C₃₋₆cycloalkyl;
each occurrence of R² is independently selected from halogen, cyano, C₁₋₈alkyl, C₁₋₈alkoxy, haloC₁₋₈alkyl, C₃₋₆cycloalkyl, 5 membered heteroaryl, -C(O)NHR, -NHC(O)R, - S(O)₂NHR and -C≡CR;
each occurrence of R is independently selected from C₁₋₈alkyl, C₃₋₁₂cycloalkyl, and C₆₋₁₄aryl;
'm' is an integer ranging from 0 to 3, both inclusive; and
'n' is an integer ranging from 0 to 3, both inclusive;
with the proviso that 'm' and 'n' are not '0' simultaneously.

The compounds of formula (I) exhibit mPGES-1 enzyme inhibition activity and, therefore, are useful for the treatment of pain and inflammation in a variety of diseases or conditions.

The compound "*N*-(4-chloro-3-(5-oxo-1-(4-(trifluoromethyl)phenyl)-4,5-dihydro-1*H-*1,2,4-triazol-3-yl)benzyl)pivalamide", herein after designated as "compound of formula (II)" is disclosed as Example 100 in the specification of WO 2013/186692. Also disclosed therein, is the process for preparation of compound of formula (II). The structural representation of compound of formula (II) is

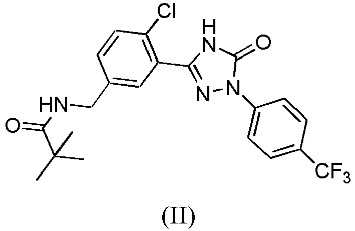

Development of a commercial drug candidate involves many steps, such as development of a cost effective synthetic method which is efficient in large scale manufacturing process. Also, in formulation of drug compositions, it is important for the active pharmaceutical ingredient to be in a form in which it can be conveniently handled and processed. Convenient handling is important not only from the perspective of obtaining a commercially viable manufacturing process, but also from the perspective of subsequent manufacture of pharmaceutical formulations comprising the active pharmaceutical ingredient. The drug development therefore involves research regarding finding suitable pharmaceutically acceptable salt forms of a drug. It may be also desirable to explore various polymorphs of the active pharmaceutical ingredient, which display better handling properties as well as it may also show improved physicochemical as well as pharmacokinetic and pharmacodynamics properties.

### SUMMARY OF THE INVENTION

The present application relates to novel solid state forms of compound of formula (II). Further the present application relates to an improved process for the manufacture of compound of formula (II) which is suitable for large scale synthesis. The process of the present application involves less number of steps and also provides the compound of formula (II) in high yield and with high purity.

In one aspect, the present application relates to a crystalline form of *N*-(4-chloro-3-(5-oxo-1-(4-(trifluoromethyl)phenyl)-4,5-dihydro-1*H*-1,2,4-triazol-3-yl)benzyl)pivalamide, represented by formula (II), having a characteristic X-ray diffraction pattern comprising peaks at 5.66, 6.55, and 13.05 ± 0.2° 2θ.

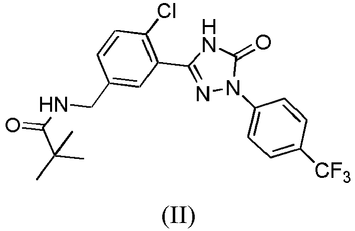

*N*-(4-chloro-3-(5-oxo-1-(4-(trifluoromethyl)phenyl)-4,5-dihydro-1*H*-1,2,4-triazol-3-yl)benzyl)pivalamide, herein after designated as compound of formula (II).

The solid state forms of compound of formula (II) exist in an anhydrous and/or solvent-free form or as a hydrate and/or a solvate form. The present invention relates to crystalline form of compound of formula (II).

The present invention relates to crystalline form of compound of formula (II) which is designated as Form I.

The present invention also relates to process for preparing crystalline form of compound of formula (II) which is designated as Form I.

The disclosure relates to crystalline form of compound of formula (II) which is designated as Form II.

The disclosure relates to process for preparing crystalline form of compound of formula (II) which is designated as Form II.

The disclosure relates to crystalline form of compound of formula (II) which is designated as Form III.

The disclosure relates to process for preparing crystalline form of compound of formula (II) which is designated as Form III.

In an embodiment of the present invention, the crystalline form of the invention is in substantially pure crystalline form. For the purpose of the present invention, the term "substantially pure" as used herein includes reference to crystalline forms of, or greater than, 90%, more preferably 95%, more preferably 97%, more preferably 99% polymorphic purity as determined, for example by X-ray powder diffraction, Raman spectroscopy or IR spectroscopy.

The disclosure relates to crystalline form of compound of formula (II), which is a mixture of any two or more forms of compound of formula (II), designated as Form I, Form II and Form III.

The disclosure provides an amorphous form of *N*-(4-chloro-3-(5-oxo-1-(4-(trifluoromethyl)phenyl)-4,5-dihydro-1*H*-1,2,4-triazol-3-yl)benzyl) pivalamide (compound of formula (II)).

The disclosure relates to process for preparing compound of formula (II) in amorphous form.

A variant of the disclosure not forming part of the invention provides a method for treating diseases, conditions and/or disorders modulated by mPGES-1 enzyme; comprising administering a crystalline form of compound of formula (II), or a pharmaceutical composition that comprises the crystalline form of compound of formula (II) along with pharmaceutically acceptable excipients.

A variant of the disclosure not forming part of the invention provides a method for treating diseases, conditions and/or disorders modulated by mPGES-1 enzyme; comprising administering a crystalline form of compound of formula (II) designated as Form (I), or a pharmaceutical composition that comprises the crystalline form of compound of formula (II) designated as Form (I), along with pharmaceutically acceptable excipients.

A variant of the disclosure not forming part of the invention provides a method for treating diseases, conditions and/or disorders modulated by mPGES-1 enzyme; comprising administering a crystalline form of compound of formula (II) designated as Form (II), or a pharmaceutical composition that comprises the crystalline form of compound of formula (II) designated as Form (II), along with pharmaceutically acceptable excipients.

A variant of the disclosure not forming part of the invention, provides a method for treating diseases, conditions and/or disorders modulated by mPGES-1 enzyme; comprising administering a crystalline form of compound of formula (II) designated as Form (III), or a pharmaceutical composition that comprises the crystalline form of compound of formula (II) designated as Form (III), along with pharmaceutically acceptable excipients.

In another embodiment, there is provided crystalline compound of formula (II) having an average particle size value (D₅₀) in the range from about 1 µm to about 100 µm.

In yet another embodiment, there is provided the crystalline compound of formula (II) having an average particle size value (D₅₀) in range from about 1 µm to about 50 µm.

In yet another embodiment, there is provided crystalline compound of formula (II) having an average particle size value (D₅₀) in the range from about 1 µm to about 20 µm.

In another embodiment, there is provided crystalline compound of formula (II) having a D₁₀ value in the range from about 0.3 µm to about 10 µm.

In yet another embodiment, there is provided crystalline compound of formula (II) having a D₁₀ value in the range from about 0.5 µm to about 8 µm.

In yet another embodiment, there is provided crystalline compound of formula (II) having a D₁₀ value in the range from about 0.5 µm to about 5 µm.

In another embodiment, there is provided crystalline compound of formula (II) having a D₉₀ value in the range from about 4 µm to about 300 µm.

In yet another embodiment, there is provided crystalline compound of formula (II) having a D₉₀ value in the range from about 5 µm to about 250 µm.

In yet another embodiment, there is provided crystalline compound of formula (II) having a D₉₀ value in the range from about 5 µm to about 200 µm.

In another aspect, there is provided a process for the preparation of a crystalline form of N-(4-chloro-3-(5-oxo-1-(4-(trifluoromethyl)phenyl)-4,5-dihydro-1H-1,2,4-triazol-3-yl)benzyl) pivalamide, having a characteristic X-ray diffraction pattern comprising peaks at 5.66, 6.55, and 13.05 ± 0.2 °2θ, the process comprising:
(a) taking *N*-(4-chloro-3-(5-oxo-1-(4-(trifluoromethyl)phenyl)-4,5-dihydro-1*H-*1,2,4-triazol-3-yl)benzyl)pivalamide in a mixture of diethyl ether and methanol or in a mixture of methyl tert-butyl ether and methanol;
(b) stirring the mixture of step (a); and
(c) isolating the desired compound.

In an embodiment there is provided substantially pure *N*-(4-chloro-3-(5-oxo-1-(4-(trifluoromethyl)phenyl)-4,5-dihydro-1*H*-1,2,4-triazol-3-yl)benzyl)pivalamide (compound of formula (II)). For the purpose of the present invention, the term "substantially pure" as used herein includes reference to purity of, or greater than, 98%, more preferably 99%, more preferably 99.5%, more preferably 99.9% purity as determined, for example by HPLC.

In yet another embodiment, the present invention relates to substantially pure compound of formula (II) having purity greater than about 98% by HPLC.

In yet another embodiment, the present invention relates to substantially pure compound of formula (II) having purity greater than about 99% by HPLC.

In yet another embodiment, the present invention relates to substantially pure compound of formula (II) having purity greater than about 99.9% by HPLC.

In yet another embodiment, three is provided compound of formula (II) which is substantially free from the impurity represented by compound of formula (II').

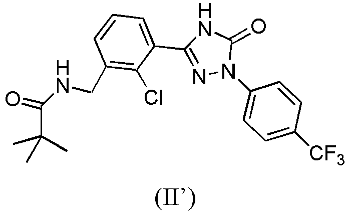

For the purpose of this invention, the term "substantially free" as used herein includes reference to presence of impurity of, or less than, 2%, more preferably 1%, more preferably 0.5%, more preferably 0.1% impurity as determined, for example by HPLC.

In an embodiment, there is provided an improved process for preparation of compound of formula (II) or its pharmaceutically acceptable salts.

In an embodiment, the present application relates to process for preparation of compound of formula (II) or salt thereof 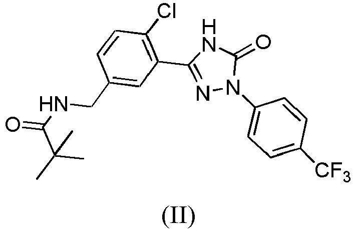 wherein the compound of formula (II) is prepared from compound of formula (III)

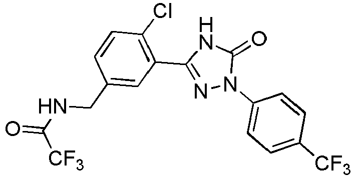

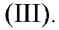

In yet another embodiment, there is provided process for preparation of compound of formula (II) or salt thereof, which process comprising the step of reacting compound of formula (IV) 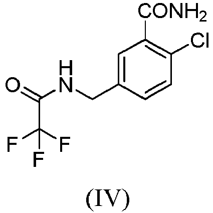 with oxalyl chloride and compound of formula (V) 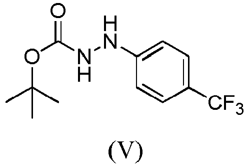 to obtain compound of formula (III)

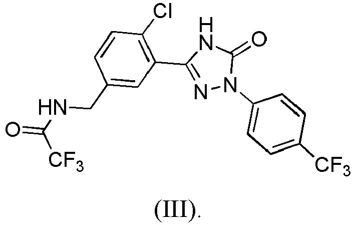

In yet another embodiment there is provided process for the preparation of compound of formula (II) or salt thereof, which process comprises the steps of :
a) reacting 2-chloro benzoic acid with 2,2,2-trifluoro-*N*-(hydroxymethyl)acetamide to obtain substantially pure compound of formula (VI); 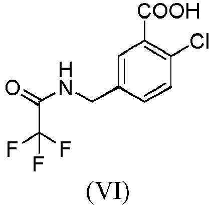
b) converting compound of formula (VI) to compound of formula (IV); 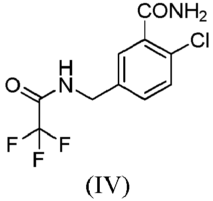
c) reacting compound of formula (IV) with oxalyl chloride and compound of formula (V) 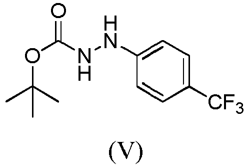 to obtain compound of formula (III); 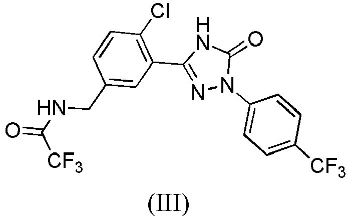 and
d) converting compound of formula (III) to compound of formula (II).

In another embodiment, there is provided process for preparation of compound of formula (II) or salt thereof, which process comprising the step of reacting 2-chloro benzoic acid with a compound (VII) 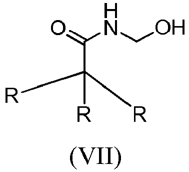 to obtain compound of formula (VIa) 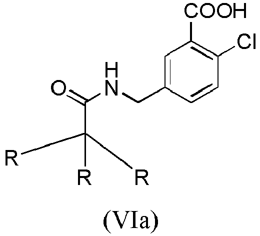 wherein R is chloro, bromo or methyl. In this embodiment, compound (VII) can be selected from 2,2,2-Trichloro-N-(hydroxymethyl)acetamide, 2,2,2-Tribromo-N-(hydroxymethyl) acetamide or 2,2,2-Trimethyl-N-(hydroxymethyl)acetamide.

### BRIEF DESCRIPTION OF THE FIGURES

FIG.1 is powder X-ray diffraction pattern of crystalline form of *N*-(4-chloro-3-(5-oxo-1-(4-(trifluoromethyl)phenyl)-4,5-dihydro-1*H*-1,2,4-triazol-3-yl)benzyl)pivalamide, designated as Form I.
FIG.2 is Infra-Red (IR) spectra of crystalline form of *N*-(4-chloro-3-(5-oxo-1-(4-(trifluoromethyl)phenyl)-4,5-dihydro-1*H*-1,2,4-triazol-3-yl)benzyl)pivalamide, designated as Form I.
FIG.3 is powder X-ray diffraction pattern of crystalline form of *N*-(4-chloro-3-(5-oxo-1-(4-(trifluoromethyl)phenyl)-4,5-dihydro-1*H*-1,2,4-triazol-3-yl)benzyl)pivalamide, designated as Form II.
FIG.4 is Infra-Red (IR) spectra of crystalline form of *N*-(4-chloro-3-(5-oxo-1-(4-(trifluoromethyl)phenyl)-4,5-dihydro-1*H*-1,2,4-triazol-3-yl)benzyl)pivalamide, designated as Form II.
FIG.5 is powder X-ray diffraction pattern of crystalline form of *N*-(4-chloro-3-(5-oxo-1-(4-(trifluoromethyl)phenyl)-4,5-dihydro-1*H*-1,2,4-triazol-3-yl)benzyl)pivalamide, designated as Form III.
FIG.6 is Infra-Red (IR) spectra of crystalline form of *N*-(4-chloro-3-(5-oxo-1-(4-(trifluoromethyl)phenyl)-4,5-dihydro-1*H*-1,2,4-triazol-3-yl)benzyl)pivalamide, designated as Form III.
FIG.7 is powder X-ray diffraction pattern of amorphous form of *N*-(4-chloro-3-(5-oxo-1-(4-(trifluoromethyl)phenyl)-4,5-dihydro-1*H*-1,2,4-triazol-3-yl)benzyl)pivalamide.

### DETAILED DESCRIPTION OF THE INVENTION

In one aspect, the present application relates to a crystalline form of *N*-(4-chloro-3-(5-oxo-1-(4-(trifluoromethyl)phenyl)-4,5-dihydro-1*H*-1,2,4-triazol-3-yl)benzyl)pivalamide, represented by formula (II), having a characteristic X-ray diffraction pattern comprising peaks at 5.66, 6.55, and 13.05 ± 0.2° 2θ.

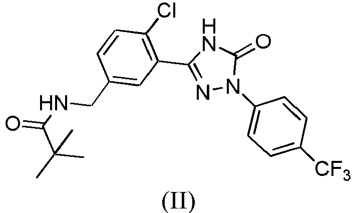

The present invention relates to solid state form of compound of formula (II) which is crystalline.

The present invention relates to crystalline form compound of formula (II) which is designated as Form I.

The present invention also relates to a process for preparing crystalline form of compound of formula (II) which is designated as Form I.

The disclosure relates to crystalline form of compound of formula (II) which is designated as Form II.

The disclosure relates to process for preparing crystalline form of compound of formula (II) which is designated as Form II.

The disclosure relates to crystalline form of compound of formula (II) which is designated as Form III.

The disclosure relates to process for preparing crystalline form of compound of formula (II) which is designated as Form III.

In an embodiment of the present invention, the crystalline form of the invention is in substantially pure crystalline form. For the purpose of the present invention, the term "substantially pure" as used herein includes reference to crystalline forms of, or greater than, 90%, more preferably 95%, more preferably 97%, more preferably 99% polymorphic purity as determined, for example by X-ray powder diffraction, Raman spectroscopy or IR spectroscopy.

The disclosure provides *N*-(4-chloro-3-(5-oxo-1-(4-(trifluoromethyl)phenyl)-4,5-dihydro-1*H*-1,2,4-triazol-3-yl)benzyl)pivalamide (compound of formula (II)) in amorphous form.

The disclosure relates to process for preparing compound of formula (II) in amorphous form.

In an embodiment there is provided substantially pure *N*-(4-chloro-3-(5-oxo-1-(4-(trifluoromethyl)phenyl)-4,5-dihydro-1*H*-1,2,4-triazol-3-yl)benzyl)pivalamide (compound of formula (II)). For the purpose of the present invention, the term "substantially pure" as used herein includes reference to purity of, or greater than, 98%, more preferably 99%, more preferably 99.5%, more preferably 99.9% purity as determined, for example by HPLC.

In yet another embodiment, the present invention relates to substantially pure compound of formula (II) having purity greater than about 98% by HPLC.

In yet another embodiment, the present invention relates to substantially pure compound of formula (II) having purity greater than about 99% by HPLC.

In yet another embodiment, the present invention relates to substantially pure compound of formula (II) having purity greater than about 99.9% by HPLC.

In yet another embodiments provided compound of formula (II) which is substantially free from the impurity represented by compound of formula (II').

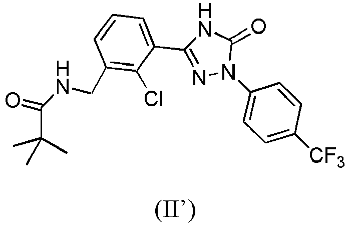

For the purpose of this invention, the term "substantially free" as used herein includes reference to presence of impurity of, or less than, 2%, more preferably 1%, more preferably 0.5%, more preferably 0.1% impurity as determined, for example by HPLC.

In another embodiment, there is provided crystalline compound of formula (II) having an average particle size value (D₅₀) in the range from about 1 µm to about 100 µm.

In yet another embodiment, there is provided the crystalline compound of formula (II) having an average particle size value (D₅₀) in range from about 1 µm to about 50 µm.

In yet another embodiment, there is provided crystalline compound of formula (II) having an average particle size value (D₅₀) in the range from about 1 µm to about 20 µm.

In another embodiment, there is provided crystalline compound of formula (II) having a D₁₀ value in the range from about 0.3 µm to about 10 µm.

In yet another embodiment, there is provided crystalline compound of formula (II) having a D₁₀ value in the range from about 0.5 µm to about 8 µm.

In yet another embodiment, there is provided crystalline compound of formula (II) having a D₁₀ value in the range from about 0.5 µm to about 5 µm.

In another embodiment, there is provided crystalline compound of formula (II) having a D₉₀ value in the range from about 4 µm to about 300 µm.

In yet another embodiment, there is provided crystalline compound of formula (II) having a D₉₀ value in the range from about 5 µm to about 250 µm.

In yet another embodiment, there is provided crystalline compound of formula (II) having a D₉₀ value in the range from about 5 µm to about 200 µm.

In yet another embodiment, there is provided the compound of formula (II) having a D₉₀ value in the range from about 5 µm to about 150 µm.

The particle size characteristic for compound of formula (II) for some of the batches is provided in Table 1.

**Table 1:**

| Batch. No | Particle size | | |
|---|---|---|---|
| | d (0.1) µm | d (0.5) µm | d (0.9) µm |
| 1 | 0.7 | 3.7 | 12.7 |
| 2 | 0.6 | 2.0 | 9.0 |
| 3 | 0.7 | 3.3 | 14.8 |
| 4 | 0.7 | 2.4 | 10.7 |
| 5 | 0.7 | 2.2 | 9.4 |

The present invention relates to crystalline form of compound of formula (II) which is designated as Form I.

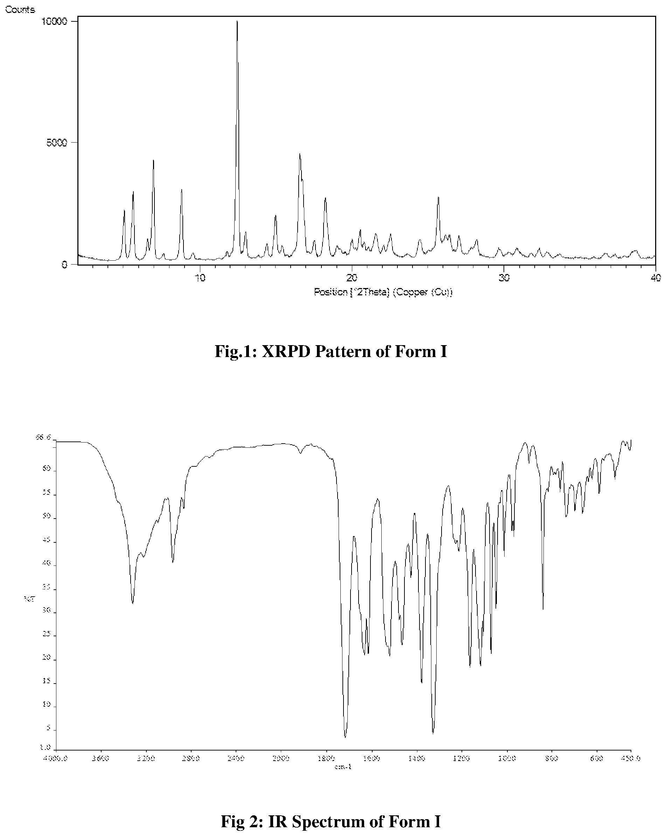

Form I of compound of formula (II) is characterized by the X-Ray Powder Diffraction (XRPD) pattern as shown in FIG. 1.

Form I of compound of formula (II) is further characterized by the characteristic X-ray diffraction pattern comprising of the following peaks expressed in terms of 2θ ±0.2: 5.66, 6.55 and 13.05.

In an embodiment, Form I of compound of formula (II) is further characterized by the characteristic X-ray diffraction pattern comprising of the following peaks expressed in terms of 2θ ±0.2: 5.66, 6.55, 12.51, 13.05, 15.01, 16.59 and 25.69.

In yet another embodiment, Form I of compound of formula (II) is further characterized by the characteristic X-ray diffraction pattern peaks expressed in terms of 2θ as presented in Table 2.

**Table 2: Prominent two theta positions and relative intensities of XRPD of Form I of compound of formula (II)**

| Angle (2θ ±0.2) | Relative intensity (%) | Angle (2θ ±0.2) | Relative intensity (%) |
|---|---|---|---|
| 5.07 | 21.13 | 18.28 | 24.69 |
| 5.66 | 28.35 | 20.00 | 7.05 |
| 6.55 | 7.22 | 20.53 | 11.23 |
| 6.99 | 41.72 | 21.59 | 9.41 |
| 8.82 | 29.52 | 22.55 | 9.42 |
| 12.51 | 100.00 | 24.46 | 7.20 |
| 13.05 | 11.12 | 25.69 | 24.98 |
| 14.48 | 6.01 | 26.14 | 9.03 |
| 15.01 | 17.96 | 26.45 | 9.27 |
| 16.59 | 42.13 | 27.07 | 8.83 |
| 16.81 | 29.64 | 28.23 | 7.56 |
| 17.56 | 6.69 | | |

In yet another embodiment, Form I of compound of formula (II) is characterized by the Fourier Transform Infrared Spectroscopy (FT-IR) pattern as shown in FIG. 2.

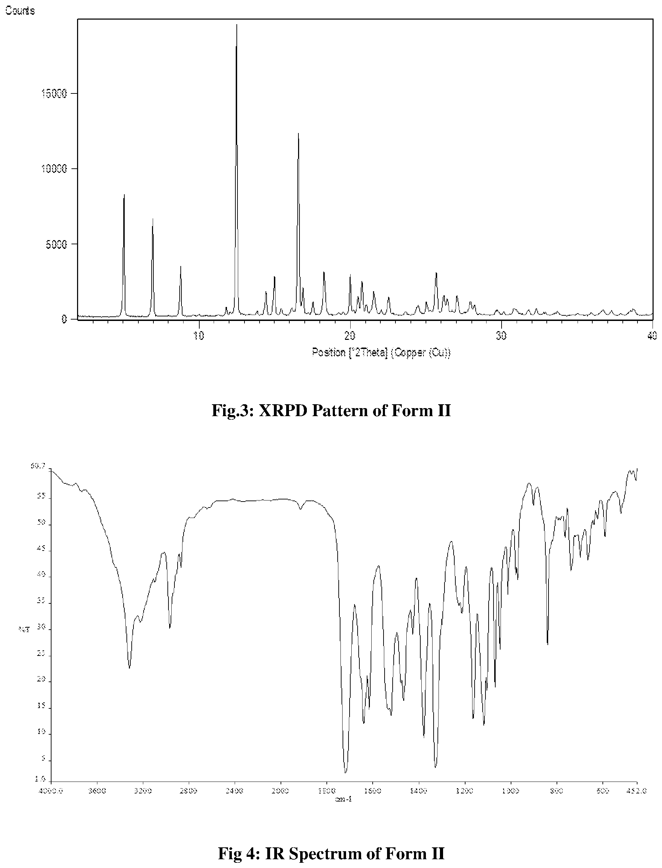

Form II, which falls outside the scope of the invention, of compound of formula (II) is characterized by the X-Ray Powder Diffraction (XRPD) pattern as shown in FIG. 3.

Form II, of compound of formula (II) is further characterized by the characteristic X-ray diffraction pattern comprising of the following peaks expressed in terms of 2θ ±0.2: 24.57 and 30.85.

Form II, , of compound of formula (II) is further characterized by the characteristic X-ray diffraction pattern comprising of the following peaks expressed in terms of 2θ ±0.2: 7.02, 12.54, 16.64, 18.31, 24.57, 25.74 and 30.85.

Form II of compound of formula (II) is further characterized by the characteristic X-ray diffraction pattern peaks expressed in terms of 2θ as presented in Table 3.

**Table 3: Prominent two theta positions and relative intensities of XRPD of Form II, which falls outside the scope of the invention. of compound of formula (II)**

| Angle (2θ ±0.2) | Relative intensity (%) | Angle (2θ ±0.2) | Relative intensity (%) |
|---|---|---|---|
| 5.07 | 13.40 | 21.59 | 10.93 |
| 7.02 | 22.82 | 22.59 | 11.52 |
| 8.85 | 12.97 | 24.44 | 9.45 |
| 12.54 | 79.59 | 24.57 | 10.01 |
| 14.50 | 10.18 | 25.06 | 5.63 |
| 15.05 | 14.36 | 25.74 | 34.24 |
| 16.64 | 100.00 | 26.22 | 13.36 |
| 16.92 | 13.77 | 26.43 | 7.29 |
| 18.31 | 27.64 | 27.07 | 9.21 |
| 20.03 | 20.28 | 27.97 | 7.49 |
| 20.56 | 15.10 | 28.25 | 7.44 |
| 20.83 | 17.87 | 30.85 | 5.62 |

Form II of compound of formula (II) is characterized by the Fourier Transform Infrared Spectroscopy (FT-IR) pattern as shown in FIG. 4.

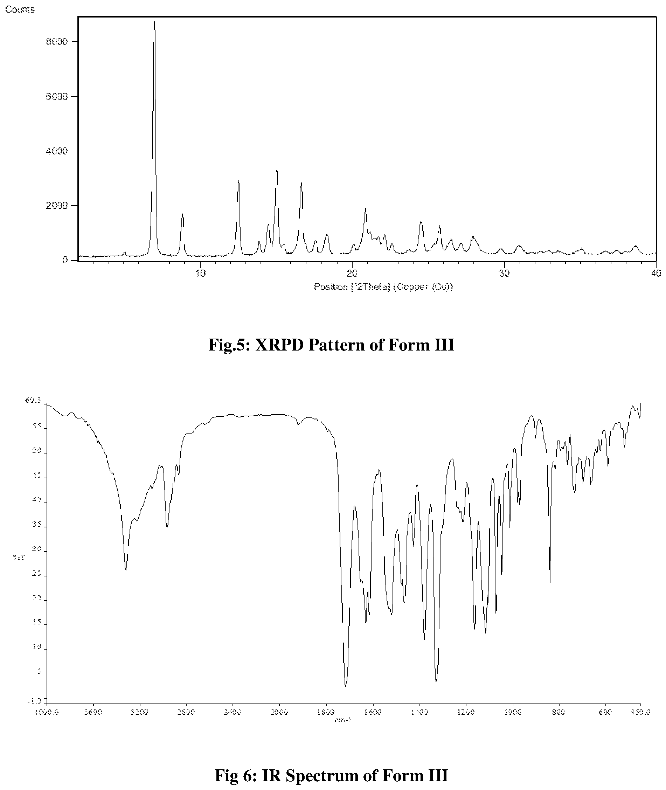

Form III, which falls outside the scope of the invention, of compound of formula (II) is characterized by the X-Ray Powder Diffraction (XRPD) pattern as shown in FIG. 5.

Form III of compound of formula (II) is further characterized by the characteristic X-ray diffraction pattern comprising of the following peaks expressed in terms of 2θ ±0.2:, 13.92, 31.01, 35.13 and 38.54.

Form III of compound of formula (II) is further characterized by the characteristic X-ray diffraction pattern comprising of the following peaks expressed in terms of 2θ ±0.2: 7.00, 12.60, 13.92, 16.71, 20.89, 24.55, 31.01, 35.13 and 38.54.

Form III of compound of formula (II) is further characterized by the characteristic X-ray diffraction pattern peaks expressed in terms of 2θ as presented in Table 4.

**Table 4: Prominent two theta positions and relative intensities of XRPD of Form III of compound of formula (II)**

| Angle (2θ ±0.2) | Relative intensity (%) | Angle (2θ ±0.2) | Relative intensity (%) |
|---|---|---|---|
| 7.00 | 100.00 | 22.69 | 6.91 |
| 8.86 | 21.61 | 23.65 | 3.55 |
| 12.60 | 37.24 | 24.55 | 20.52 |
| 13.92 | 11.19 | 25.29 | 5.10 |
| 14.53 | 21.77 | 25.83 | 8.92 |
| 15.10 | 64.50 | 26.55 | 9.56 |
| 16.71 | 41.21 | 27.18 | 6.86 |
| 17.62 | 11.10 | 27.94 | 15.23 |
| 18.31 | 8.42 | 31.01 | 5.22 |
| 20.89 | 48.85 | 35.13 | 6.07 |
| 21.70 | 13.65 | 38.54 | 5.67 |
| 22.16 | 12.85 | | |

Form III of compound of formula (II) is characterized by the Fourier Transform Infrared Spectroscopy (FT-IR) pattern as shown in FIG. 6.

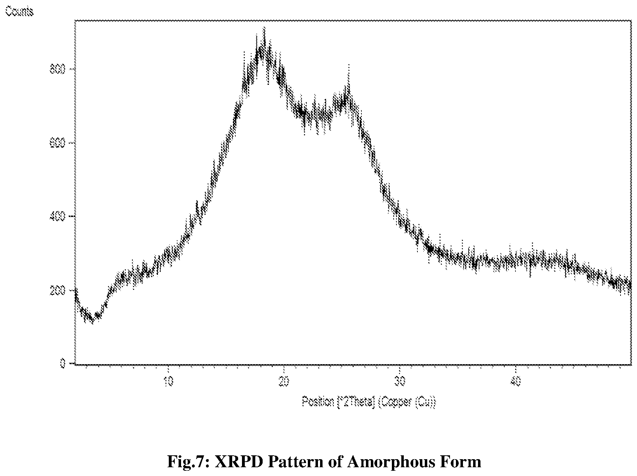

The disclosure relates to an amorphous form of compound of formula (II).An amorphous form, which falls outside the scope of the invention, of compound of formula (II) is characterized by the X-Ray Powder Diffraction (XRPD) pattern as shown in FIG. 7.

In another aspect, the present invention relates to process for preparing crystalline form of compound of formula (II) which is designated as Form I.

The process to prepare Form I comprises taking the compound of formula (II) in a mixture of diethyl ether and methanol. Alternatively, the compound of formula (II) can be taken in a mixture of methyl tert-butyl ether and methanol. Preferably, the compound of formula (II) can be taken in the mixture of solvent under inert atmosphere. The reaction mass may be stirred at a suitable temperature for a suitable period of time. The suitable temperature may be about 20 to 40°C, preferably about 25 to 30°C. The suitable period may be up to 5 hours, preferably about 1 -2 hours. The solid may be collected by methods including decantation, centrifugation, gravity filtration, suction filtration, or any other technique for the isolation of solids. In a preferred embodiment, the solid may be filtered and washed with diethyl ether or methyl tert-butyl ether.

The recovered solid may be optionally further dried. Drying may be carried out in a tray dryer, vacuum oven, air oven, fluidized bed drier, spin flash dryer, flash dryer, or the like. The drying may be carried out at atmospheric pressure or under a reduced pressure at suitable temperatures as long as the compound of formula (II) is not degraded in quality. The drying may be carried out for any desired time until the required purity limit of LOD is achieved. For example, it may vary from about 1 to about 10 hours or longer.

A process for preparing crystalline form of compound of formula (II) which is designated as Form II, which does not form part of the invention, is described below.

The process to prepare Form II comprises heating the compound of formula (II) to melt by external heating source such as heating mantle. The temperature for heating may be about 150 - 300 °C, preferably about 200°C. The reaction mass may be cooled slowly to a suitable temperature to obtain crystalline form of compound of formula (II) which is designated as Form II. The suitable temperature to which the reaction mass may be cooled may be in the temperature range of 20 - 50°C, preferably 25 - 30°C.

Alternatively, crystalline form of compound of formula (II) which is designated as Form II can be obtained by the process, which process comprises taking the compound of formula (II) in ethyl acetate. The compound of formula (II) may also be taken in a mixture of ethyl acetate and methanol. Preferably, the compound of formula (II) can be taken in the solvent or mixture of solvents under inert atmosphere. The reaction mass may be stirred at a suitable temperature for a suitable period of time. The suitable temperature may be about 20 to 40°C, preferably about 25 to 30°C. The suitable period may be up to 5 hours, preferably about 1 -2 hours. The solid may be collected by methods including decantation, centrifugation, gravity filtration, suction filtration, or any other technique for the isolation of solids. The recovered solid may be optionally further dried. Drying may be carried out in a tray dryer, vacuum oven, air oven, fluidized bed drier, spin flash dryer, flash dryer, or the like. The drying may be carried out at atmospheric pressure or under a reduced pressure at suitable temperatures as long as the compound of formula (II) is not degraded in quality. The drying may be carried out for any desired time until the required purity is achieved. For example, it may vary from about 1 to about 10 hours or longer.

A process for preparing crystalline form of compound of formula (II) which is designated as Form III, which does not form part of the invention, is described below.

The process to prepare Form III comprises taking the compound of formula (II) in tetrahydrofuran. Preferably, the compound of formula (II) can be taken in the solvent under inert atmosphere. The reaction mass may be stirred at a suitable temperature. The suitable temperature may be about 20 to 40°C, more preferably about 25 to 30°C. The reaction mass may be stirred for a suitable period of time. The suitable period may be up to 1 hour, preferably about 10 - 15 minutes. To the reaction mixture, a non-polar solvent may be added. The non-polar solvent may be any hydrocarbon solvent such as n-heptane, n-hexane or n-pentane. The reaction mixture may be further stirred at a suitable temperature for suitable period of time. The suitable temperature may be about 20 to 40°C, more preferably about 25 to 30°C. The suitable period may be up to 10 hours, preferably about 3-4 hours. The solid may be collected by methods including decantation, centrifugation, gravity filtration, suction filtration, or any other technique for the isolation of solids. The solid may be filtered and washed with n-heptane. The isolated solid may be optionally further dried. Drying may be carried out in a tray dryer, vacuum oven, air oven, fluidized bed drier, spin flash dryer, flash dryer, or the like. The drying may be carried out at atmospheric pressure or under a reduced pressure at suitable temperatures as long as the compound of formula (II) is not degraded in quality. The drying may be carried out for any desired time until the required purity limit of LOD is achieved. For example, it may vary from about 1 to about 10 hours or longer.

A process for preparing compound of formula (II) in amorphous form, which does not form part of the invention, is described below.

The process comprises heating the compound of formula (II) to melt by external heating device such as heating mantle. The temperature for heating may be about 150 - 300°C, preferably about 200°C. The reaction mass may be cooled rapidly to a suitable temperature to obtain the compound of formula (II) in amorphous form. The suitable temperature to which the compound may be cooled may be in the temperature range of 20 - 50°C, preferably 25 - 30°C.

The disclosure provides a pharmaceutical composition comprising an excipients, carriers, diluents or mixture thereof, and therapeutically acceptable amount of crystalline compound of formula (II) designated as Form (I).

The disclosure provides a method of treating diseases, conditions and/or disorders modulated by mPGES-1 enzyme; comprising administering a crystalline form of compound of formula (II) designated as Form (I), or a pharmaceutical composition that comprises the crystalline form of compound of formula (II) designated as form (I) along with pharmaceutically acceptable excipients.

The disclosure provides a pharmaceutical composition comprising an excipients, carriers, diluents or mixture thereof, and therapeutically acceptable amount of crystalline compound of formula (II) designated as Form (II).

The disclosure provides a method of treating diseases, conditions and/or disorders modulated by mPGES-1 enzyme; comprising administering a crystalline form of compound of formula (II) designated as Form (II), or a pharmaceutical composition that comprises the crystalline form of compound of formula (II) designated as form (II) along with pharmaceutically acceptable excipients.

The disclosure provides a pharmaceutical composition comprising an excipients, carriers, diluents or mixture thereof, and therapeutically acceptable amount of crystalline compound of formula (II) designated as Form (III).

The disclosure provides a method of treating diseases, conditions and/or disorders modulated by mPGES-1 enzyme; comprising administering a crystalline form of compound of formula (II) designated as Form (III), or a pharmaceutical composition that comprises the crystalline form of compound of formula (II) designated as form (III) along with pharmaceutically acceptable excipients.

The disclosure provides a method of treating diseases or conditions or disorders associated with mPGES-1 enzyme, which are selected from inflammation, asthma, chronic obstructive pulmonary disease, pulmonary fibrosis, inflammatory bowel disease, irritable bowel syndrome, pain, inflammatory pain, chronic pain, acute pain, fever, migraine, headache, low back pain, fibromyalgia, myofascial disorders, viral infections, influenza, common cold, herpes zoster, hepatitis C, AIDS, bacterial infections, fungal infections, dysmenorrhea, burns, surgical or dental procedures, malignancies hyperprostaglandin E syndrome, classic Bartter syndrome, synovitis, atherosclerosis, gout, arthritis, osteoarthritis, juvenile arthritis, rheumatoid arthritis, juvenile onset rheumatoid arthritis, rheumatic fever, ankylosing spondylitis, Hodgkin's disease, systemic lupus erythematosus, vasculitis, pancreatitis, nephritis, bursitis, conjunctivitis, iritis, scleritis, uveitis, wound healing, dermatitis, eczema, psoriasis, stroke, diabetes mellitus, cancer, neurodegenerative disorders such as Alzheimer's disease, Parkinson's disease, Amyotrophic lateral sclerosis and multiple sclerosis, autoimmune diseases, allergic disorders, rhinitis, ulcers, mild to moderately active ulcerative colitis, familial adenomatous polyposis, coronary heart disease, and sarcoidosis by administering crystalline compound of formula (II) designated as Form (I), Form (II) or Form (III).

The disclosure provides a method of treating diseases or conditions or disorders associated with mPGES-1 enzyme, which is selected from of pain, chronic pain, acute pain, rheumatoid arthritis pain or osteoarthritic pain by administering crystalline compound of formula (II), designated as Form (I), Form (II) or Form (III), in therapeutically effective amount.

The disclosure provides a method of treating pain, chronic pain, acute pain, rheumatoid arthritis pain or osteoarthritic pain by administering crystalline compound of formula (II), designated as Form (I), Form (II) or Form (III), in therapeutically effective amount.

The disclosure provides a method of treating inflammation, neurodegenerative disorders such as Parkinson's disease, Alzheimer's disease and amyotrophic lateral sclerosis by administering crystalline compound of formula (II), designated as Form (I), Form (II) or Form (III), in therapeutically effective amount.

In another embodiment, there is provided process for the preparation of compound of formula (II) which process comprises the steps as shown in the scheme 1.

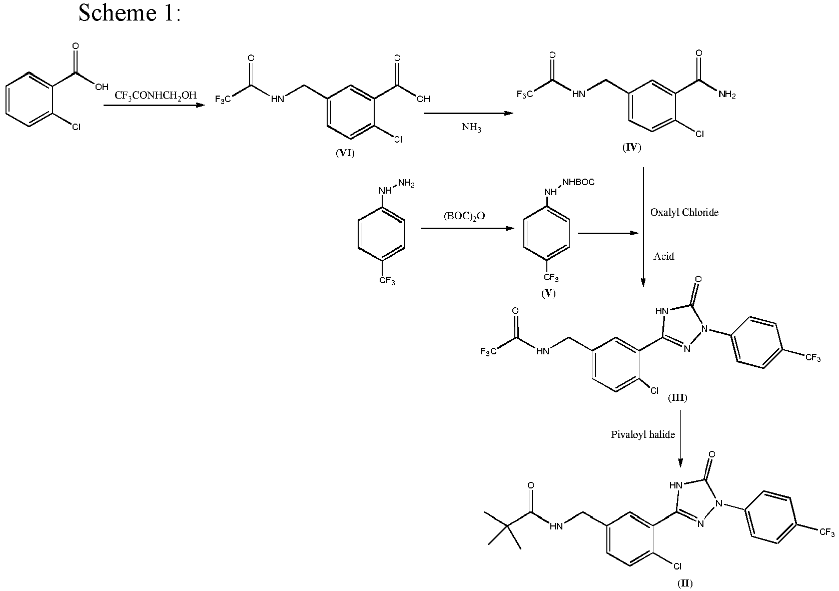

The process to prepare compound of formula (II) comprises:
a) reacting 2-chloro benzoic acid with 2,2,2-trifluoro-*N*-(hydroxymethyl)acetamide to obtain substantially pure compound of formula (VI); 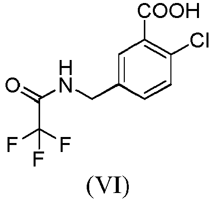
b) converting compound of formula (VI) to compound of formula (IV); 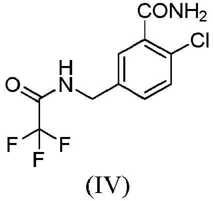
c) reacting compound of formula (IV) with oxalyl chloride and compound of formula (V) 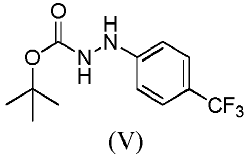 to obtain compound of formula (III) 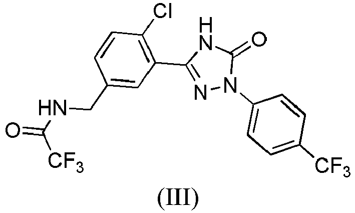 and
d) converting compound of formula (III) to compound of formula (II)

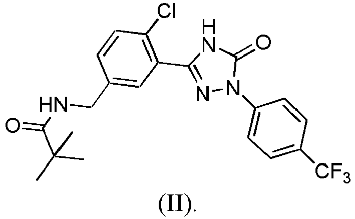

Step (a) involves reacting 2-chloro benzoic acid with 2,2,2-trifluoro-*N-*(hydroxymethyl)acetamide to obtain compound of formula (VI). The reaction may be carried out in presence of an acid. The acid may be inorganic or organic acid. Preferably, the acid may be inorganic acid. More preferably, the acid is sulphuric acid (H₂SO₄). The reaction may be carried out at a suitable temperature. The suitable temperature may be about 20 - 50 °C, preferably 25 - 30 °C.

After the completion of the reaction, the reaction mass may be poured into water to obtain a solid product, which may be filtered and dried. The solid product may be further purified by taking the product in a suitable solvent or mixture of solvents thereof. The suitable solvent may include, but not limited to, hydrocarbon solvents such as toluene, xylene, n-heptane, cyclohexane and n-hexane and/or ether solvent such as methyl ethyl ketone. Preferably, the suitable solvent(s) is toluene and/or methyl ethyl ketone. The mixture may be optionally heated till a clear solution is obtained. The clear solution may be cooled to obtain the solid which may be filtered and dried. The purification step may be further repeated to obtain a product of desired purity.

Step (b) involves converting compound of formula (VI) to compound of formula (IV). The compound of formula (VI) is treated with an activating agent to form its activated carboxylic acid derivative, followed by treatment with a source of ammonia to obtain compound of formula (IV).

In an embodiment, the compound of formula (VI) may be first treated with an activating agent to form its activated carboxylic acid derivative. The activating agent includes, but not limited to, oxalyl chloride, thionyl chloride, phosphorous trichloride, phosphorous pentachloride and the like. Preferably the activating agent is oxalyl chloride. The reaction of compound of formula (VI) with an activating agent may be catalysed by adding a suitable reagent such as DMF or triethyl amine. In an embodiment, the catalysing reagent is DMF. The reaction may be carried out in a suitable solvent. The suitable solvent may be a polar solvent. Preferably the solvent is tetrahydofuran. In another embodiment, the reaction mixture may be stirred at a suitable temperature for a suitable period of time. The suitable temperature may be about 25 - 30 °C. The suitable time period may be 2 - 4 hours. After the completion of the reaction, the reaction mass may be subjected to evaporation to distil out the excess of solvent to obtain activated carboxylic acid derivative of compound of formula (VI).

The activated carboxylic acid derivative of compound of formula (VI) may be further treated with a source of ammonia to obtain compound of formula (IV). The source of ammonia includes, but not limited to, formamide, ammonia gas, aqueous ammonia, alcoholic ammonia and the like. Preferably the source of ammonia is ammonia gas. The reaction may be carried out in any polar solvent. Preferably the solvent is tetrahydofuran. In an embodiment, the ammonia gas may be purged in the reaction mixture. The reaction mixture may be stirred at a suitable temperature for a suitable period of time. The suitable temperature may be about 25 - 30 °C. The suitable period of time may be 1 - 2 hours. The reaction mixture may be maintained at a suitable pH. The suitable pH may be about 7 - 9.

After the completion of the reaction, the reaction may be quenched with water, and the reaction mass may be extracted with a suitable solvent. The suitable solvent for extraction may be ethyl acetate. The organic layer may be dried and evaporated to dryness to obtain a product. The product obtained may be taken in a non-polar solvent and stirred. The non-polar solvent may be any hydrocarbon solvent. Preferably the solvent is cyclohexane. The product may be filtered and dried to obtain the compound of formula (IV).

Step (c) involves reacting compound of formula (IV) with oxalyl chloride and compound of formula (V) to obtain compound of formula (III).

The compound of formula (IV) may be first reacted with oxalyl chloride. The reaction may be carried out in a halogenated solvent. The halogenated solvent may be methylene dichloride, ethylene dichloride, chloroform or carbon tetrachloride. Preferably the halogenated solvent is methylene dichloride. The reaction mixture may be heated to a suitable temperate for a suitable period of time. The suitable temperature may be about 45 - 50 °C. The suitable time period may be 4 - 24 hours. The excess solvent in the reaction mixture may be evaporated under the reduced pressure to obtain a residue. A solution of compound of formula (V) in a halogenated solvent may be added to the residue. The halogenated solvent may methylene dichloride, ethylene dichloride, chloroform or carbon tetrachloride. Preferably the halogenated solvent is methylene dichloride. The reaction mixture may be stirred at a suitable temperature for a suitable period of time. In another embodiment, the temperature of the reaction may be about 15 - 20 °C. In yet another embodiment the reaction may be stirred for about 4 - 6 hours.

The reaction mixture may be further treated with an acid. The acid may be an organic acid. Preferably the organic acid is trifluoroacetic acid. The reaction mixture may be stirred at a suitable temperature for a suitable period of time. The suitable temperature may be about 25 - 30 °C. The suitable time period may be about 15 - 20 hours. After the completion of the reaction, a suitable solvent may be added to the reaction mixture. The suitable solvent may be a non-polar solvent. The non-polar solvent may be any hydrocarbon solvent. Preferably the hydrocarbon solvent is cyclohexane. The reaction mixture may be maintained at a suitable temperate for a suitable period of time. The suitable temperature may be about 25 - 30 °C. The suitable time period may be 1 - 2 hours. The precipitate obtained may be filtered and dried to obtain compound of formula (III). Optionally, the product may be further taken in a suitable solvent to obtain a clear solution. The suitable solvent may be any polar solvent. Preferably, the polar solvent is tetrahydrofuran. The temperature range for the process may be about 65 - 70°C. Another suitable non-polar solvent may be charged to the clear solution. The suitable solvent may be a non-polar solvent. The non-polar solvent may be any hydrocarbon solvent. Preferably the hydrocarbon solvent is cyclohexane. The solution may be cooled to room temperature and the product precipitated may be filtered and washed with a suitable non-polar solvent such as cyclohexane to obtain compound of formula (III). The purification step may be further repeated to obtain the product of desired purity.

The compound of formula (V) may be prepared by reacting 4-(trifluoromethyl)phenylhydrazine or its suitable salt such as hydrochloride with di *tert* butyl dicarbonate. The hydrazine compound may be taken in a suitable solvent or mixture of solvents in which the compound is soluble. The suitable solvent may be water and polar solvent. Preferably the polar solvent is tetrahydrofuran. The reaction may be carried out in presence of mixture of water and tetrahydrofuran. The reaction may be carried out in the presence of a suitable base. The suitable base may be any inorganic base. The inorganic base may be sodium carbonate, potassium carbonate or lithium carbonate. Preferably, the inorganic base is sodium carbonate.

The reaction mixture may be stirred at a suitable temperature for a suitable period of time. The suitable temperature of the reaction may be room temperature (25 - 30 °C). The suitable period of time may be about 5 - 7 hours. The reaction mixture may be extracted with suitable solvent. The suitable solvent may be ethyl acetate. The organic layer may be dried and evaporated to dryness to obtain a product. The product obtained may be taken in a non-polar solvent. The non-polar solvent may be any hydrocarbon solvent. Preferably the solvent is cyclohexane. The product may be filtered and dried to obtain compound of formula (V). Optionally the product may be further washed with another solvent such as pentane to obtain the desired purity.

Step (d) involves reacting compound of formula (III) with pivaloyl halide to obtain compound of formula (II). In an embodiment, compound of formula (III) may be reacted with pivaloyl chloride. The reaction may be carried out in the presence of a suitable base. The suitable base may be any inorganic base. The inorganic base may be sodium hydroxide, potassium hydroxide or lithium hydroxide. Preferably, the inorganic base is sodium hydroxide. The reaction may be carried out in a suitable solvent or mixture of solvents. The suitable solvent may be water and any polar solvent. Preferably the polar solvent is tetrahydrofuran. The reaction may be carried out in presence of mixture of tetrahydrofuran and water.

In an embodiment, the compound of formula (III) may be first treated with the base at a suitable temperature. The suitable temperature may be 0 - 5 °C. The reaction mass be stirred at a suitable temperature for a suitable period of time. The suitable temperature may be about 10 - 20 °C. The suitable period may be 1 - 2 hour(s). The reaction mixture may be cooled to a suitable temperature and pivaloyl halide is added to it slowly. The suitable temperature may be 0 - 5 °C. The reaction mass may be maintained at a suitable temperature for a suitable period of time. The suitable temperature may be about 0 - 5 °C. The suitable period may be 30 - 60 minutes. To the reaction mixture conc. HCl or 10% NaOH solution may be optionally added to adjust the pH at 6 - 7 and the reaction mixture may be further stirred. The product precipitated may be filtered to obtain compound of formula (II). Optionally, the compound of formula (II) may be purified using another suitable solvent. The suitable solvent may be IPA. The compound and solvent mixture may be heated to obtain a clear solution. The solution may be cooled, precipitate obtained may be filtered and dried to obtain compound of formula (II) with enhanced purity. The purification step may be further repeated to obtain the desired purity.

There is provided process for preparation of compound of formula (II) 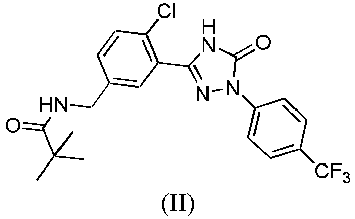 from compound of formula (III)

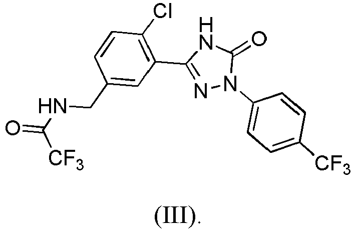

In an embodiment, there is provided process for preparation of compound of formula (II), which process comprises reacting compound of formula (III) with pivaloyl halide to obtain compound of formula (II).

In an embodiment, the compound of formula (III) may be reacted with pivaloyl chloride. The reaction may be carried out in presence of a suitable base. The suitable base may be an inorganic base. The inorganic base may be sodium hydroxide, potassium hydroxide or lithium hydroxide. Preferably, the inorganic base is sodium hydroxide. The reaction may be carried out in a suitable solvent or mixture of solvents. The suitable solvent may be water and polar solvent. Preferably the polar solvent is tetrahydrofuran. The reaction may be carried out in presence of water and tetrahydrofuran.

In another embodiment, the compound of formula (III) may be first treated with the water and base at a suitable temperature. The suitable temperature may be 0 - 5 °C. The reaction mass be stirred at a suitable temperature for a suitable period of time. The suitable temperature may be about 10 - 20 °C. The suitable period may be 1 - 2 hour(s). The reaction mixture may be cooled to a suitable temperature and pivaloyl halide may be added to it slowly. The suitable temperature may be 0 - 5 °C. The reaction mass be maintained at a suitable temperature for a suitable period of time. The suitable temperature may be about 0 - 5 °C. The suitable period may be 30 - 60 minutes. To the reaction mixture conc. HCl or 10% NaOH solution may be optionally added to adjust the pH at 6 - 7 and the reaction mixture may be further stirred. The product precipitated may be filtered and dried to obtain compound of formula (II). Optionally, the compound of formula (II) may be purified using a suitable solvent. The suitable solvent may be IPA. The compound and solvent mixture may be heated to obtain a clear solution. The solution may be cooled, precipitate obtained may be filtered and dried to obtain compound of formula (II) with enhanced purity. The purification step may be further repeated to obtain the desired purity.

There is provided a process for preparation of compound of formula (II), which process comprising reacting compound of formula (IV) 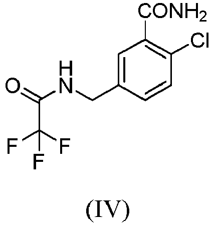 with oxalyl chloride and compound of formula (V) 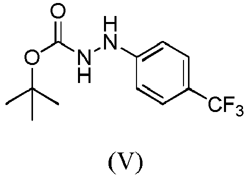 to obtain compound of formula (III).

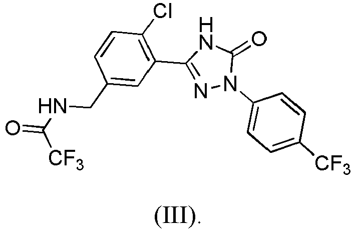

In an embodiment, the process involves reacting compound of formula (IV) with oxalyl chloride followed by reaction with compound of formula (V).

In another embodiment, the compound of formula (IV) may be first treated with oxalyl chloride. In this embodiment the reaction may be carried at temperature about 45 - 50 °C. In another embodiment, the reaction may be stirred for 4 - 5 hours. The reaction may be carried in a halogenated solvent. The halogenated solvent may methylene dichloride, ethylene dichloride, chloroform or carbon tetrachloride. Preferably the halogenated solvent is methylene dichloride. The excess solvent in the reaction mixture may be evaporated under the reduced pressure to obtain a residue. A solution of compound of formula (V) in a suitable solvent may be added to the above reaction mixture. The suitable solvent may be a halogenated solvent. The halogenated solvent may be methylene dichloride, ethylene dichloride, chloroform or carbon tetrachloride. Preferably the halogenated solvent is methylene dichloride. The reaction mixture may be further stirred at a suitable temperature for a suitable period of time. In this embodiment, the temperature of the reaction may be about 15 - 20 °C and the reaction may be stirred for about 4 - 6 hours.

The reaction mixture may be further treated with an acid. The acid may be an organic acid. Preferably the organic acid is trifluoroacetic acid. The reaction mixture may be stirred at a suitable temperature for another suitable period of time. The suitable temperature may be about 25 - 30 °C. The suitable time period may be about 15 - 20 hours. After the completion of the reaction, a suitable solvent may be added to the reaction mixture. The suitable solvent may be a non-polar solvent. The non-polar solvent may be any hydrocarbon solvent. Preferably the hydrocarbon solvent is cyclohexane. The reaction may be maintained at a suitable temperate for a suitable period of time. The suitable temperature may be about 25 - 30 °C. The suitable period of time may be 1 - 2 hours. The precipitate obtained may be filtered and dried to obtain compound of formula (III). Optionally, the product may be further taken in a suitable solvent to obtain a clear solution. The suitable solvent may be any polar solvent. Preferably, the polar solvent is tetrahydrofuran. The temperature range for the process may be 65 - 70°C. A suitable non-polar solvent may be charged to the clear solution. The suitable solvent may be a non-polar solvent. The non-polar solvent may be any hydrocarbon solvent. Preferably the hydrocarbon solvent is cyclohexane. The solution may be cooled to room temperature and the compound precipitated may be filtered and washed with a suitable non-polar solvent such as cyclohexane to obtain compound of formula (III). The purification step may be further repeated to obtain the desired purity.

The compound of formula (V) may be prepared by reacting 4-(trifluoromethyl)phenylhydrazine or its suitable salt such as hydrochloride with di *tert* butyl dicarbonate. The hydrazine compound may be taken in a suitable solvent or mixture of solvents in which the compound is soluble. The suitable solvent may be water and polar solvent. Preferably the polar solvent is tetrahydrofuran. The reaction may be carried out in presence of mixture of water and tetrahydrofuran. The reaction may be carried out in the presence of a suitable base. The suitable base may be any inorganic base. The inorganic base may be sodium carbonate, potassium carbonate or lithium carbonate. Preferably, the inorganic base is sodium carbonate.

The reaction mixture may be stirred at a suitable temperature for a suitable period of time. The suitable temperature of the reaction may be room temperature (25 - 30 °C). The suitable period of time may be about 5 - 7 hours. The reaction mixture may be extracted with suitable solvent. The suitable solvent may be ethyl acetate. The organic layer may be dried and evaporated to dryness to obtain a product. The product obtained may be taken in a non-polar solvent. The non-polar solvent may be any hydrocarbon solvent. Preferably the solvent is cyclohexane. The product may be filtered and dried to obtain compound of formula (V). Optionally the product may be further washed with another solvent such as pentane to obtain the desired purity.

In another embodiment, there is provided process for preparation of compound of formula (II) or salt thereof, which process comprising the step of reacting 2-chloro benzoic acid with a compound (VII) 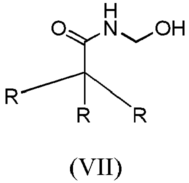 to obtain compound of formula (VI a) 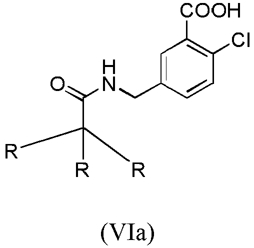 wherein R is chloro, bromo, or methyl.

In this embodiment, compound (VII) can be selected from 2,2,2-Trichloro-N-(hydroxymethyl)acetamide, 2,2,2-Tribromo-N-(hydroxymethyl) acetamide or 2,2,2-Trimethyl-N-(hydroxymethyl)acetamide. In a further embodiment the reaction may be carried out in presence of an acid. The acid may be inorganic or organic acid. Preferably, the acid may be inorganic acid. More preferably, the acid is sulphuric acid (H₂SO₄). The reaction may be carried out at a suitable temperature. The suitable temperature may be room temperature (25 - 30 °C).

After the completion of the reaction, the reaction mass may be poured in cold water to obtain a compound represent by formula (VI a), which may be filtered and dried. The compound of formula (VI a) may be further purified by taking the compound in a suitable solvent or mixture thereof and optionally heating the mixture till a clear solution is obtained. The suitable solvent may include, but not limited to, hydrocarbon solvents such as toluene, xylene, n-heptane, cyclohexane and n-hexane. Preferably, the suitable solvent is toluene and/or methyl ethyl ketone. The clear solution may be cooled to obtain the compound of formula (VI a) which may be filtered and dried. The purification step may be further repeated to obtain the desired purity.

### PHARMACEUTICAL COMPOSITIONS

The compounds of the invention are typically administered in the form of a pharmaceutical composition. Such compositions can be prepared using procedures well known in the pharmaceutical art and comprise at least one compound of the invention. The pharmaceutical composition of the present patent application comprises one or more compounds described herein and one or more pharmaceutically acceptable excipients. Typically, the pharmaceutically acceptable excipients are approved by regulatory authorities or are generally regarded as safe for human or animal use. The pharmaceutically acceptable excipients include, but are not limited to, carriers, diluents, glidants and lubricants, preservatives, buffering agents, chelating agents, polymers, gelling agents, viscosifying agents, solvents and the like.

Examples of suitable carriers include, but are not limited to, water, salt solutions, alcohols, polyethylene glycols, peanut oil, olive oil, gelatin, lactose, terra alba, sucrose, dextrin, magnesium carbonate, sugar, amylose, magnesium stearate, talc, gelatin, agar, pectin, acacia, stearic acid, lower alkyl ethers of cellulose, silicic acid, fatty acids, fatty acid amines, fatty acid monoglycerides and diglycerides, fatty acid esters, and polyoxyethylene.

The pharmaceutical composition may also include one or more pharmaceutically acceptable auxiliary agents, wetting agents, suspending agents, preserving agents, buffers, sweetening agents, flavoring agents, colorants or any combination of the foregoing.

The pharmaceutical compositions may be in conventional forms, for example, capsules, tablets, solutions, suspensions, injectables or products for topical application. Further, the pharmaceutical composition of the present invention may be formulated so as to provide desired release profile.

Administration of the compounds of the invention, in pure form or in an appropriate pharmaceutical composition, can be carried out using any of the accepted routes of administration of pharmaceutical compositions. The route of administration may be any route which effectively transports the active compound of the patent application to the appropriate or desired site of action. Suitable routes of administration include, but are not limited to, oral, nasal, buccal, dermal, intradermal, transdermal, parenteral, rectal, subcutaneous, intravenous, intraurethral, intramuscular, or topical.

Solid oral formulations include, but are not limited to, tablets, capsules (soft or hard gelatin), dragees (containing the active ingredient in powder or pellet form), troches and lozenges.

Liquid formulations include, but are not limited to, syrups, emulsions, and sterile injectable liquids, such as suspensions or solutions.

Topical dosage forms of the compounds include ointments, pastes, creams, lotions, powders, solutions, eye or ear drops, impregnated dressings, and may contain appropriate conventional additives such as preservatives, solvents to assist drug penetration.

The pharmaceutical compositions of the present patent application may be prepared by conventional techniques, e.g., as described in Remington: The Science and Practice of Pharmacy, 20th Ed., 2003 (Lippincott Williams & Wilkins).

Suitable doses of the compounds for use in treating the diseases and disorders described herein can be determined by those skilled in the relevant art. Therapeutic doses are generally identified through a dose ranging study in humans based on preliminary evidence derived from the animal studies. Doses must be sufficient to result in a desired therapeutic benefit without causing unwanted side effects. Mode of administration, dosage forms, and suitable pharmaceutical excipients can also be well used and adjusted by those skilled in the art. All changes and modifications are envisioned within the scope of the present patent application.

### DEFINITIONS

The term "crystalline" as used herein, means having a regularly repeating arrangement of molecules or external face planes.

The term "amorphous" as used herein, means essentially without regularly repeating arrangement of molecules or external face planes.

Unless stated otherwise, percentages stated throughout this specification are weight/weight (w/w) percentages.

The term "mixture" as used herein, means a combination of at least two substances, in which one substance may be completely miscible, partially miscible or essentially immiscible in the other substance.

The term "treating" or "treatment" of a state, disorder or condition includes; (a) preventing or delaying the appearance of clinical symptoms of the state, disorder or condition developing in a subject that may be afflicted with or predisposed to the state, disorder or condition but does not yet experience or display clinical or subclinical symptoms of the state, disorder or condition; (b) inhibiting the state, disorder or condition, i.e., arresting or reducing the development of the disease or at least one clinical or subclinical symptom thereof; or (c) relieving the disease, i.e., causing regression of the state, disorder or condition or at least one of its clinical or subclinical symptoms.

The term "subject" includes mammals (especially humans) and other animals, such as domestic animals (e.g., household pets including cats and dogs) and non-domestic animals (such as wildlife).

All powder X-ray diffraction patterns were obtained using: Panlytical X'PERT-PRO diffractometer model and measured with Cu-Kα1 radiation at wavelength of 1.54060 A°. The obtained powder X-ray diffraction profiles were integrated using X'Pert High Score Plus Software.

It is meant to be understood that peak heights in a powder x-ray diffraction pattern may vary and will be dependent on variables such as the temperature, crystal size, crystal habit, sample preparation or sample height in the analysis well of the Scintag×2 Diffraction Pattern System.

All FTIR spectra were recorded using KBr on Perkin-Elmer instrument (Model: Spectrum One). The data was processed using Spectrum One Software.

As used herein, the term "average particle size" (or synonymously, "mean particle size") refers to the distribution of particles, wherein about 50 volume percent of all the particles measured have a size less than the defined average particle size value and about 50 volume percent of all measurable particles measured have a particle size greater than the defined average particle size value. This can be identified by the term "D₅₀" or "d (0.5)".

The term "D₁₀" refers to the distribution of particles, wherein about 10 volume percent of all the particles measured have a size less than the defined particle size value. This can be identified by the term "d(0.1)" as well. Similarly, as used herein, the term "D₉₀" refers to the distribution of particles, wherein about 90 volume percent of all the particles measured have a size less than the defined particle size value. This can be identified by the term or "d (0.9)" as well.

The average particle size can be measured using various techniques like laser diffraction, photon correlation spectroscopy and Coulter's principle. Typically, instruments like ZETASIZER® 3000 HS (Malvern® Instruments Ltd., Malvern, United Kingdom), *NICOMP* 388™ *ZLS* system *(PSS-Nicomp* Particle Sizing Systems, Santa Barbara, CA, USA), or Coulter Counter are generally used to determine the mean particle size. Preferably, Mastersizer 2000 (Malvern® Instruments Ltd., Malvern, United Kingdom) is used to determine the particle size of the particles.

### EXPERIMENTAL

Unless otherwise stated, work-up includes distribution of the reaction mixture between the organic and aqueous phase indicated within parentheses, separation of layers and drying the organic layer over sodium sulphate, filtration and evaporation of the solvent. Purification, unless otherwise mentioned, includes purification by silica gel chromatographic techniques, generally using ethyl acetate/petroleum ether mixture of a suitable polarity as the mobile phase. Use of a different eluent system is indicated within parentheses. The following abbreviations are used in the text: DMSO-*d*₆: Hexadeuterodimethyl sulfoxide; AcOEt: ethyl acetate; equiv. or eq.: equivalents; h : hour(s); L : litres; CDCl₃: deuterated chloroform; CHCl₃ : chloroform; EtOAc or EA: ethyl acetate; DCM or MDC: dichloromethane or methylene dichloride; DMSO: dimethyl sulfoxide; DMF: *N,N*-dimethylformamide; MTBE: Methyl *tert-butyl* ether; DSC: Differential scanning calorimetry; K₂CO₃ potassium carbonate; MeOH : methanol; EtOH: ethanol; NaHCO₃: sodium bicarbonate; Na₂CO₃: sodium carbonate; THF : tetrahydrofuran; J: Coupling constant in units of Hz; RT or rt: room temperature (22-30°C); q.s.: quantity sufficient; aq.: aqueous; equiv. or eq.: equivalents; conc. : concentrated; min: minutes; i.e. : that is; h or hrs : hours.

The parameters mentioned in the description which characterize the polymorphic nature, particle size by the measuring techniques and methods are described below:

### Particle Size Distribution studies:

The particle size distribution was measured using Mastersizer 2000 (Malvern® instruments Ltd., Malvern, United Kingdom) with following measuring equipment and settings:
Instrument: Malvern Mastersizer 2000
Sample Handling Unit: Hydro 2000S (A)
Dispersant RI: 1.33
Dispersant: Water
Sample quantity: 25 - 50 mg
Measurement time: 5.0 sec

### Powder X-ray Diffraction Studies:

All powder X-ray diffraction patterns were obtained using: Panlytical X'PERT-PRO diffractometer model and measured with Cu-Kα1 radiation at wavelength of 1.54060 A°. The obtained powder X-ray diffraction profiles were integrated using X'Pert High Score Plus Software.

### Fourier Transform Infrared Spectroscopy (FT-IR) Studies

About 200 mg of KBr, previously dried at 200 °C and cooled, was taken into a mortar and grinded to a fine powder. 2-3 mg of test sample was added to it and was mixed well and grinded to obtain a uniform sample. A small quantity of the sample powder was taken, put it between dies and compressed it by applying 10-15 pound pressure to obtain a semitransparent pellet. The IR spectrum of the pellet was recorded from 4000 cm⁻¹ to 450 cm⁻¹ taking air as a reference.

### HPLC Conditions:

Apparatus: A High Performance Liquid Chromatograph equipped with quaternary gradient pumps, variable wavelength UV detector attached with data recorder and Integrator software or equivalent.
Column: Zorbax Eclipse XDB C-8, 150 mm × 4.6 mm, 5 µ or equivalent
Mobile phase: A: Buffer B: Methanol (Gradient Program)
Buffer : Water, pH adjusted to 2.2 with trifluoroacetic acid solution. Filter through 0.45µ filter paper and degas.
Diluent: Water: Methanol (20:80 v/v)
Flow Rate: 1.0 mL/minute
Detection wavelength: UV 270 nm
Column temperature: 25°C
Injection volume: 20µl
Run time: 60 min

### Test solution:

25 mg of the compound was weighed and transferred into a 50 mL volumetric flask. Methanol (5 ml) was added and it was sonicated to dissolve. The diluent was added to make up the solution to the mark of the flask.

### Procedure:

The equal volumes of blank (diluent) and test solution were separately injected into the High Performance liquid chromatography. The responses were recorded eliminating the peaks due to blank and the chromatographic purity by area was calculated by normalization method.

The following examples are presented to provide what is believed to be the most useful and readily understood description of procedures and conceptual aspects of this invention. The examples provided below are merely illustrative of the invention and are not intended to limit the same to disclosed embodiments.

### Intermediate 1

### Preparation of 2-chloro-5-((2,2,2-trifluoroacetamido)methyl)benzoic acid

To a solution of 2-chlorobenzoic acid (930.0 gm,5.96 mol) in conc. sulphuric acid (5580 ml), 2,2,2-trifluoro-N-(hydroxymethyl)acetamide (930.0 gm ,6.0 mol) was added at 20 °C and the reaction mixture was stirred for 15.0 h at 25-30 °C. After the completion of the reaction, the reaction was slowly quenched with cold water (37.0 lit) at 10-15 °C. The precipitated product was filtered and dried. The obtained product was purified using mixture of toluene and methylethylketone (7:1 x 4 times) to obtain 580.0 gm of the tittle compound having chemical purity of 98.0%, determined by HPLC.

### Intermediate 2

### Preparation of 2-chloro-5-((2,2,2-trifluoroacetamido)methyl)benzamide

To a solution of 2-chloro-5-((2,2,2-trifluoroacetamido)methyl)benzoic acid (575.0 gm, 2.04 mol) in THF (3450.0 ml) catalytic amount of DMF (2.9 ml) was added. Oxalyl chloride (389.0 gm, 3.06 mol) was added to the reaction mixture slowly at 20 °C. The reaction mixture was stirred for 2.0 h at 25-30 °C. After the completion of the reaction, the reaction mass was distilled under reduced pressure to obtain the acid chloride derivative. THF (3450.0 ml) was added and the reaction mixture was cooled to 0-5 °C. Ammonia gas was purged to the reaction mixture till a pH of 7-9 was obtained and the reaction mixture was stirred for 1.0 h at 20-25 °C. After the completion of the reaction, the reaction was quenched with water (7.0 lit). The reaction mass was extracted with ethyl acetate (2×7.0 lit). The organic layer was washed with brine. The organic layer was concentrated under reduced pressure. Cyclohexane (1440 ml) was charged to the residue and the mixture was maintained for 1.0 h at 25-30 °C. The product precipitated was filtered and dried to obtain 505.0 gm of the title compound having chemical purity of 95.0%, determined by HPLC.

### Intermediate 3

### Preparation of tert-butyl 2-(4-(trifluoromethyl)phenyl)hydrazinecarboxylate

To a solution of 4-(trifluoromethyl)phenyl]hydrazine HCl (400.0 gm,1.88 mol) in THF (2400 ml), water (1200 ml) was added. Sodium carbonate (480 gm, 4.52 mol) was added to the reaction mixture slowly. The reaction mass was cooled to 10-15 °C and *di-tert-*butyl dicarbonate (596 gm, 2.73 mol) was added slowly. The reaction mixture was stirred for 6.0 h at 25-30 °C. After the completion of the reaction, the reaction was quenched with water (15.0 lit) and the reaction mass was extracted with ethyl acetate (2×5.0 lit). The organic layer was washed with water followed by brine. The organic layer was concentrated under reduced pressure. Cyclohexane (1.0 lit) was charged to the obtained residue and it was stirred for 1.0 h at 25-30 °C. The precipitated product was filtered and dried to obtain 480.0 gm of the title compound having chemical purity of 99%, determined by HPLC.

### Intermediate 4

### Preparation of N-(4-chloro-3-(5-oxo-1-(4-(trifluoromethyl)phenyl)-4,5-dihydro-1H-1,2,4-triazol-3-yl)benzyl)-2,2,2-trifluoroacetamide

A solution of 2-chloro-5-((2,2,2-trifluoroacetamido)methyl)benzamide (500 gm, 1.78 mol) in MDC (5.0 lit) was cooled to 20 °C and oxalyl chloride (295 gm, 2.32 mol) was added to it slowly. The reaction mixture was stirred at 45-50 °C for 4.0 h. The reaction mass was concentrated under reduced pressure. A solution of *tert-butyl* 2-[4-(trifluoromethyl)phenyl]hydrazinecarboxylate (492 gm, 1.78 mmol) in MDC (6.0 lit) was prepared separately and was added to the above residue at 15-20 °C. The reaction mixture was stirred for 4.0 h. Trifluoroacetic acid (600.0 ml) was added to the reaction mixture at 10-15 °C and the reaction was stirred for 15.0 h at 25-30 °C. After the completion of the reaction, cyclohexane (12.0 lit) was added to the reaction mixture and it was maintained for 1.0 h at 25-30 °C. The product precipitated was filtered and dried to obtain 734.0 gm of the title compound having chemical purity of 98%, determined by HPLC.

In the following examples, examples 1 and 2 directed to form (I) are illustrative of the present invention while the further examples 2-6 including forms (II) and (III) and an amorphous form are provided as reference examples.

### Example 1

### Preparation of N-(4-chloro-3-(5-oxo-1-(4-(trifluoromethyl)phenyl)-4,5-dihydro-1H-1,2,4-triazol-3-yl)benzyl)pivalamide

To a solution of *N*-(4-chloro-3-(5-oxo-1-(4-(trifluoromethyl)phenyl)-4,5-dihydro-1*H-*1,2,4-triazol-3-yl)benzyl)-2,2,2-trifluoroacetamide (35.0 gm, 0.075 mol) in THF (175.0 ml) water (280.0 ml) was added and the reaction mixture was cooled to 5 °C. Sodium hydroxide solution (10.55 gm, 0.263 mol) in water (70.0 ml) was added slowly to the reaction mixture and it was stirred for 1.0 h at 15-20 °C. The reaction mixture was cooled to 5 °C and pivaloyl chloride (13.6 gm, 0.113 mol) was added slowly. The reaction mixture was stirred for 30.0 min at same temperature. The pH of the reaction mixture was adjusted to about 6 - 7 by adding 20 % HCl or 20.0% sodium hydroxide solution and the reaction mixture was maintained for another 30.0 min at 10-15 °C. The product precipitated was filtered and dried to obtain 29.0 gm of the title compound having chemical purity of 99.5%, determined by HPLC.

### Example 2

### Preparation of Form (I) of N-(4-chloro-3-(5-oxo-1-(4-(trifluoromethyl)phenyl)-4,5-dihydro-1H-1,2,4-triazol-3-yl)benzyl)pivalamide

### Method 1:

*N*-(4-chloro-3-(5-oxo-1-(4-(trifluoromethyl)phenyl)-4,5-dihydro-1*H-*1,2,4-triazol-3-yl)benzyl)pivalamide (29.0 gm) was dissolved in 15% methanol in ethyl acetate (1450 ml) at 45 °C and the reaction mixture was concentrated under the reduced pressure. To the residue obtained, 10.0% methanol in MTBE (290.0 ml) was charged and the mixture was stirred for 1.0 h at 25-30 °C. The mixture was filtered and the filtrate was dried to obtain 25.0 gm of Form (I) of *N-*(4-chloro-3-{5-oxo-1-[4-(trifluoromethyl)phenyl]-4,5-dihydro-1*H*-1,2,4-triazol-3-yl} benzyl)-pivalamide.

### Method 2:

*N*-(4-chloro-3-(5-oxo-1-(4-(trifluoromethyl)phenyl)-4,5-dihydro-1*H*-1,2,4-triazol-3-yl) benzyl)pivalamide (10.0 gm) was taken in a mixture of diethyl ether (60 ml) and methanol (30 ml) and the reaction mixture was stirred for 2 h. The mixture was filtered and the solid was dried to obtain 7.5 gm of Form (I) of *N*-(4-chloro-3-{5-oxo-1-[4-(trifluoromethyl)phenyl]-4,5-dihydro-1*H*-1,2,4-triazol-3-yl}benzyl)-pivalamide.

### Reference example 3

### Preparation of Form (II) of N-(4-chloro-3-(5-oxo-1-(4-(trifluoromethyl)phenyl)-4,5-dihydro-1H-1,2,4-triazol-3-yl)benzyl)pivalamide

### Method 1:

*N*-(4-chloro-3-(5-oxo-1-(4-(trifluoromethyl)phenyl)-4,5-dihydro-1*H*-1,2,4-triazol-3-yl) benzyl)pivalamide (1.0 gm) was heated to melt by external heating. The mass was cooled to room temperature (25 - 30 °C) slowly to obtain 0.90 gm of Form (II) of *N*-(4-chloro-3-{5-oxo-1-[4-(trifluoromethyl)phenyl]-4,5-dihydro-1*H*-1,2,4-triazol-3-yl}benzyl)-pivalamide.

### Method 2:

*N*-(4-chloro-3-(5-oxo-1-(4-(trifluoromethyl)phenyl)-4,5-dihydro-1*H*-1,2,4-triazol-3-yl) benzyl)pivalamide (1.0 gm) was crystallized in ethyl acetate (5 ml) and methanol (5 ml) to obtain 0.60 gm of Form (II) of *N*-(4-chloro-3-{5-oxo-1-[4-(trifluoromethyl)phenyl]-4,5-dihydro-1*H*-1,2,4-triazol-3-yl}benzyl)-pivalamide.

### Method 3:

*N*-(4-chloro-3-(5-oxo-1-(4-(trifluoromethyl)phenyl)-4,5-dihydro-1*H*-1,2,4-triazol-3-yl) benzyl)pivalamide (1.0 gm) was slurred in ethyl acetate (3 ml) to obtain 0.75 gm of Form (II) of *N*-(4-chloro-3-{5-oxo-1-[4-(trifluoromethyl)phenyl]-4,5-dihydro-1*H*-1,2,4-triazol-3-yl}benzyl)-pivalamide

### Reference example 4

### Preparation of Form (III) of N-(4-chloro-3-(5-oxo-1-(4-(trifluoromethyl)phenyl)-4,5-dihydro-1H-1,2,4-triazol-3-yl)benzyl)pivalamide

*N*-(4-chloro-3-(5-oxo-1-(4-(trifluoromethyl)phenyl)-4,5-dihydro-1*H*-1,2,4-triazol-3-yl) benzyl)pivalamide (10.0 gm) was taken in THF (150 ml). The mixture was stirred for 10-15 min at 25-30 °C to obtain clear solution. n-Heptane (500 ml) was added to the clear solution at a moderate speed in 5-10 min. A solid was precipitated which was further stirred for 3 - 4 h. The mixture was filtered, the solid was washed with n-heptane (50 ml) and it was dried to obtain 8.3 gm of Form (III) of *N*-(4-chloro-3-{5-oxo-1-[4-(trifluoromethyl)phenyl]-4,5-dihydro-1*H*-1,2,4-triazol-3-yl}benzyl)-pivalamide.

### Reference example 5

### Preparation of amorphous form of N-(4-chloro-3-(5-oxo-1-(4-(trifluoromethyl)phenyl)-4,5-dihydro-1H-1,2,4-triazol-3-yl)benzyl)pivalamide

*N*-(4-chloro-3-(5-oxo-1-(4-(trifluoromethyl)phenyl)-4,5-dihydro-1*H*-1,2,4-triazol-3-yl) benzyl)pivalamide (1.0 gm) was heated to melt by external heating. The mass was cooled to room temperature (25 - 30 °C) rapidly to obtain 0.80 gm of amorphous form of *N*-(4-chloro-3-{5-oxo-1-[4-(trifluoromethyl)phenyl]-4,5-dihydro-1*H*-1,2,4-triazol-3-yl}benzyl)-pivalamide (>95%) .

### Reference example 6

### Stability studies of compound of formula II: (N-(4-chloro-3-(5-oxo-1-(4-(trifluoromethyl) phenyl)-4,5-dihydro-1H-1,2,4-triazol-3-yl)benzyl)pivalamide):

The compound of formula II was stored under conditions as shown in below table and a total amount of degradation products (related substances) as well as single maximum impurity formed during storage was estimated by HPLC. The material was packed in inner clear polythene bag under nitrogen lined with black polythene bag, covered with triple laminated aluminium bag placed in HDPE drum and subjected to the conditions mentioned in the table 5.

**Table 5:**

| Storage conditions | Condition 1 | Condition 2 |
|---|---|---|
| Temperature | 25 ± 2 °C | 40 ± 2 °C |
| Humidity (%RH) | 60 ± 5 % RH | 75 ± 5 % RH |
| Testing intervals | 0,1,2,3, 6 months | 0,1,2,3, 6 months |

The result of stability studies of compound of formula (II) carried out under the conditions mentioned in the table 5 is presented in tables 6 and 7.

### Stability of compound of formula II under the storage condition 1:

**Table 6:**

| Test Item (%) | Storage period (months) | | | | |
|---|---|---|---|---|---|
| | 0 | 1 | 2 | 3 | 6 |
| Related substances | 0.13 | NT | NT | 0.14 | 0.14 |
| Single max. impurity | 0.13 | NT | NT | 0.14 | 0.14 |

| | | | | | |
|---|---|---|---|---|---|
| NT: not tested | | | | | |

### Stability of compound of formula II under the storage condition 2:

**Table 7:**

| Test Item (%) | Storage period (months) | | | | |
|---|---|---|---|---|---|
| | 0 | 1 | 2 | 3 | 6 |
| Related substances | 0.13 | 0.14 | 0.13 | 0.14 | 0.13 |
| Single max. impurity | 0.13 | 0.14 | 0.13 | 0.14 | 0.13 |

Although the invention herein has been described with reference to particular embodiments, it is to be understood that these embodiments are merely illustrative of the principles and applications of the present invention.

## Claims

1. A crystalline form of *N*-(4-chloro-3-(5-oxo-1-(4-(trifluoromethyl)phenyl)-4,5-dihydro-1*H-*1,2,4-triazol-3-yl)benzyl)pivalamide, having a characteristic X-ray diffraction pattern comprising peaks at 5.66, 6.55, and 13.05 ± 0.2° 2θ.

2. The crystalline form according to claim 1, having a characteristic X-ray diffraction pattern further comprising peaks at 12.51, 15.01, 16.59 and 25.69 ± 0.2° 2θ.

3. The crystalline form according to claim 1, having an average particle size value (D₅₀) in the range from about 1 µm to about 100 µm.

4. The crystalline form according to claim 1, having an average particle size value (D₅₀) in range from about 1 µm to about 50 µm.

5. The crystalline form according to claim 1, having a D₁₀ value in the range from about 0.3 µm to about 10 µm.

6. The crystalline from according to claim 1, having a D₁₀ value in the range from about 0.5 µm to about 8 µm.

7. The crystalline form according to claim 1, having a D₉₀ value in the range from about 4 µm to about 300 µm.

8. The crystalline form according to claim 1, having a D₉₀ value in the range from about 5 µm to about 250 µm.

9. The crystalline form according to claim 1, having a D₉₀ value in the range from about 5 µm to about 200 µm.

10. The crystalline form according to claim 1, wherein the polymorphic purity of the crystalline form is greater than 90%.

11. The crystalline form according to claim 1, wherein the polymorphic purity of the crystalline form is greater than 95%.

12. The crystalline form according to claim 1, wherein the polymorphic purity of the crystalline form is greater than 97%.

13. The crystalline form according to claim 1, wherein the polymorphic purity of the crystalline form is greater than 99%.

14. A process for the preparation of a crystalline form of N-(4-chloro-3-(5-oxo-1-(4-(trifluoromethyl)phenyl)-4,5-dihydro-1H-1,2,4-triazol-3-yl)benzyl) pivalamide, having a characteristic X-ray diffraction pattern comprising peaks at 5.66, 6.55, and 13.05 ± 0.2 °2θ, the process comprising:
(a) taking *N*-(4-chloro-3-(5-oxo-1-(4-(trifluoromethyl)phenyl)-4,5-dihydro-1*H*-1,2,4-triazol-3-yl)benzyl)pivalamide in a mixture of diethyl ether and methanol or in a mixture of methyl *tert-butyl* ether and methanol;
(b) stirring the mixture of step (a); and
(c) isolating the desired compound.

## Patentansprüche

1. Kristalline Form von *N*-(4-Chlor-3-(5-oxo-1-(4-(trifluormethyl)phenyl)-4,5-dihydro-1*H-*1,2,4-triazol-3-yl)benzyl)pivalamid, die ein charakteristisches Röntgenbeugungsmuster aufweist, das Peaks bei 5,66, 6,55 und 13,05 ± 0,2 ° 2θ umfasst.

2. Kristalline Form nach Anspruch 1, die ein charakteristisches Röntgenbeugungsmuster aufweist, das ferner Peaks bei 12,51, 15,01, 16,59 und 25,69 ± 0,2 ° 2θ umfasst.

3. Kristalline Form nach Anspruch 1, die einen durchschnittlichen Partikelgrößenwert (D₅₀) in dem Bereich von etwa 1 µm bis etwa 100 µm aufweist.

4. Kristalline Form nach Anspruch 1, die einen durchschnittlichen Partikelgrößenwert (D₅₀) in dem Bereich von etwa 1 µm bis etwa 50 µm aufweist.

5. Kristalline Form nach Anspruch 1, die einen Wert D₁₀ in dem Bereich von etwa 0,3 µm bis etwa 10 µm aufweist.

6. Kristalline Form nach Anspruch 1, die einen Wert D₁₀ in dem Bereich von etwa 0,5 µm bis etwa 8 µm aufweist.

7. Kristalline Form nach Anspruch 1, die einen Wert D₉₀ in dem Bereich von etwa 4 µm bis etwa 300 µm aufweist.

8. Kristalline Form nach Anspruch 1, die einen Wert D₉₀ in dem Bereich von etwa 5 µm bis etwa 250 µm aufweist.

9. Kristalline Form nach Anspruch 1, die einen Wert D₉₀ in dem Bereich von etwa 5 µm bis etwa 200 µm aufweist.

10. Kristalline Form nach Anspruch 1, wobei die polymorphe Reinheit der kristallinen Form größer als 90 % ist.

11. Kristalline Form nach Anspruch 1, wobei die polymorphe Reinheit der kristallinen Form größer als 95 % ist.

12. Kristalline Form nach Anspruch 1, wobei die polymorphe Reinheit der kristallinen Form größer als 97 % ist.

13. Kristalline Form nach Anspruch 1, wobei die polymorphe Reinheit der kristallinen Form größer als 99 % ist.

14. Verfahren zur Herstellung einer kristallinen Form von N-(4-Chlor-3-(5-oxo-1-(4-(trifluormethyl)phenyl)-4,5-dihydro-1H-1,2,4-triazol-3-yl)benzyl)pivalamid, die ein charakteristisches Röntgenbeugungsmuster aufweist, das Peaks bei 5,66, 6,55 und 13,05 ± 0,2 ° 2θ umfasst, wobei das Verfahren Folgendes umfasst:
(a) Nehmen von *N*-(4-Chlor-3-(5-oxo-1-(4-(trifluormethyl)phenyl)-4,5-dihydro-1*H-*1,2,4-triazol-3-yl)benzyl)pivalamid in einem Gemisch aus Diethylether und Methanol oder in einem Gemisch aus Methyl-*tert*-butylether und Methanol;
(b) Rühren des Gemischs aus Schritt (a); und
(c) Isolieren der gewünschten Verbindung.

## Revendications

1. Forme cristalline de *N*-(4-chloro-3-(5-oxo-1-(4-(trifluorométhyl)phényl)-4,5-dihydro-1*H*-1,2,4-triazol-3-yl)benzyl)pivalamide, ayant un profil de diffraction des rayons X caractéristique comprenant des pics à 5,66, 6,55, et 13,05 ± 0,2° 2θ.

2. Forme cristalline selon la revendication 1, ayant un profil de diffraction des rayons X caractéristique comprenant en outre des pics à 12,51, 15,01, 16,59 et 25,69 ± 0,2° 2θ.

3. Forme cristalline selon la revendication 1, ayant une valeur de taille moyenne des particules (D₅₀) comprise dans la plage d'environ 1 µm à environ 100 µm.

4. Forme cristalline selon la revendication 1, ayant une valeur de taille moyenne des particules (D₅₀) comprise dans la plage d'environ 1 µm à environ 50 µm.

5. Forme cristalline selon la revendication 1, ayant une valeur D₁₀ comprise dans la plage d'environ 0,3 µm à environ 10 µm.

6. Forme cristalline selon la revendication 1, ayant une valeur D₁₀ comprise dans la plage d'environ 0,5 µm à environ 8 µm.

7. Forme cristalline selon la revendication 1, ayant une valeur D₉₀ comprise dans la plage d'environ 4 µm à environ 300 µm.

8. Forme cristalline selon la revendication 1, ayant une valeur D₉₀ comprise dans la plage d'environ 5 µm à environ 250 µm.

9. Forme cristalline selon la revendication 1, ayant une valeur D₉₀ comprise dans la plage d'environ 5 µm à environ 200 µm.

10. Forme cristalline selon la revendication 1, dans laquelle la pureté polymorphe de la forme cristalline est supérieure à 90 %.

11. Forme cristalline selon la revendication 1, dans laquelle la pureté polymorphe de la forme cristalline est supérieure à 95 %.

12. Forme cristalline selon la revendication 1, dans laquelle la pureté polymorphe de la forme cristalline est supérieure à 97 %.

13. Forme cristalline selon la revendication 1, dans laquelle la pureté polymorphe de la forme cristalline est supérieure à 99 %.

14. Procédé destiné à la préparation d'une forme cristalline de N-(4-chloro-3-(5-oxo-1-(4-(trifluorométhyl)phényl)-4,5-dihydro-1H-1,2,4-triazol-3-yl)benzyl) pivalamide, ayant un profil de diffraction des rayons X caractéristique comprenant des pics à 5,66, 6,55, et 13,05 ± 0,2° 2θ, le procédé comprenant :
(a) la prise de *N*-(4-chloro-3-(5-oxo-1-(4-(trifluorométhyl)phényl)-4,5-dihydro-1*H*-1,2,4-triazol-3-yl)benzyl)pivalamide dans un mélange d'éther diéthylique et de méthanol ou dans un mélange d'éther méthyl tert-butylique et de méthanol ;
(b) l'agitation du mélange de l'étape (a) ; et
(c) l'isolation du composé souhaité.
